# EUROPEAN PATENT APPLICATION

(11) **EP 0 699 688 A2**
(43) Date of publication of application: **06.03.1996**
(21) Application number: 95112207.6
(22) Date of filing: 03.08.1995
(51) Int. Cl.: C07K 16/12, C12N 5/20, C12P 21/08, G01N 33/577, G01N 33/569

(54) **Chlamydia pneumoniae specific monoclonal antibodies and their diagnostic use**

(30) Priority: 03.08.1994 JP 182240/94; 03.08.1994 JP 182241/94; 28.10.1994 JP 264307/94; 08.06.1995 JP 141721/95
(71) Applicant: HITACHI CHEMICAL CO., LTD., Tokyo 163-04 (JP)
(72) Inventor: Kuroiwa, Yasuyuki, Hitachinaka-shi, Ibaraki 312 (JP); Bamba, Kenzo, Hitachinaka-shi, Ibaraki 312 (JP); Kawagoe, Kiyotaka, Hitachi-shi, Ibaraki 316 (JP); Iguchi, Akifumi, Hitachi-shi, Ibaraki 317 (JP); Morikawa, Toshihide, 2-chome, Tsuchiura-shi, Ibaraki 300 (JP); Izutsu, Hiroshi, Tsukuba-shi, Ibaraki 300-32 (JP); Nakao, Yoshiki, Hitachi-shi, Ibaraki 317 (JP); Yamaki, Mitsuo, Mito-shi, Ibaraki 310 (JP); Nagayama, Akemi, Takahagi-shi, Ibaraki 318 (JP); Nakano, Hiroko, Hitachi-shi, Ibaraki 319-14 (JP); Saito, Shigeru, Hitachi-shi, Ibaraki 316 (JP)
(74) Representative: VOSSIUS & PARTNER

(57) **Abstract**

This invention relates to a monoclonal antibody which has a specific reactivity to the major outer membrane protein and the elementary body that originate from *C*. *pneumoniae* but which does not have any reactivity to the major outer membrane protein of *C*. *trachomatis*; a monoclonal antibody which has a reactivity to the 53 K dalton antigen protein of *C*. *pneumoniae* but which does not have any reactivity to the inclusion body of *C*. *trachomatis* nor *C*. *psittaci*; methods for preparing these monoclonal antibodies; cell lines producing the same; methods for detecting and determining *C*. *pneumoniae* using the same; reagents for detecting and determining *C*. *pneumoniae* containing the same; methods for diagnosis of *C*. *pneumoniae* infections using the same; and diagnostic reagents for *C*. *pneumoniae* infections comprising the same as an active ingredient.

## Description

The invention relates to monoclonal antibodies having a reactivity specific to Chlamydia pneumoniae, a method for production of the monoclonal antibodies, such antibody producing cells, a method for production of the cells, a method for detection and/or measurement of Chlamydia pneumoniae, reagents for the method, a method and agents for diagnosis of Chlamydia pneumoniae infection. The invention can be effectively used in the pharmaceutical industry, particularly in the preparation of agents for diagnosing Chlamydia pneumoniae infection.

Four kinds of species are known in Chlamydia, that is, Chlamydia trachomatis, Chlamydia pneumoniae, Chlamydia psittaci and Chlamydia pecorum. Chlamydia pneumoniae is believed to cause respiratory infections, atypical pneumonia and the like.

Since the symptoms of infections in the respiratory apparatus which are caused by Chlamydia pneumoniae are similar to those of infections which are caused by Mycoplasma pneumoniae or Influenza virus, physicians often make a wrong diagnosis. Hence, there is a need for the development of a simple method for diagnosing the infections caused by Chlamydia pneumoniae.

In general, an infection can reliably be diagnosed by detecting the causative bacterium in the infectious site or by detecting an antibody against the causative bacterium in a body fluid such as a serum and the like. The former method is called an antigen test and the latter is called an antibody test. Both of them are clinically important.

The antigen test includes an indirect fluorescent antibody technique in which an antibody against the antigen to be detected is added to a sample and a secondary antibody labeled with a fluorescent agent and the like is then added to determine the presence or absence of the antigen in the sample and a direct fluorescent antibody technique in which the fluorescent secondary antibody is not used but an antibody against the antigen to be detected that is preliminarily labeled with a fluorescent agent and the like is added to a sample to determine the presence or absence of the antigen in the sample.

As antigens of Chlamydia pneumoniae, lipopolysaccharides (LPSs) and proteins are known. LPSs have an antigenicity common to genus Chlamydia. As proteinaceous antigens, 39.5 KDa, 60 KDa, 75 KDa, 98 KDa proteins and the like are known. The 39.5 KDa protein is called Major Outer Membrane Protein (hereinafter abbreviated to "MOMP").

As antigens specific to genus Chlamydia, GLXA (a genus-specific glycolipidantigen), lipopolysaccharides (LPSs), MOMPs and the like are known. Elizabeth S. Stuart et al. reported a monoclonal antibody against GLXA of genus Chlamydia (Current Microbiology, 28, 85-90 (1994)). Harland D. Caldwell et al. reported a monoclonal antibody against LPS of genus Chlamydia (Infection and Immunity, 44(2), 306-314 (1984)). They used the monoclonal antibody to stain an inclusion body of Chlamydia pneumoniae by the direct fluorescent antibody technique. Ellena M. Peterson et al. (Infection and Immunity, 59(11), 4147-4153 (1991)) and Byron E. Batteiger et al. (Infection and Immunity, 53(3), 530-533 (1986)) reported a monoclonal antibody against MOMP of genus Chlamydia.

Since the report of Richard S. Stephens et al. (J. Immunology, 128(3), 1083-1089 (1982)), there have been published many reports on monoclonal antibodies against Chlamydia trachomatis. H. Puy et al. reported monoclonal antibodies against Chlamydia psittaci which include a monoclonal antibody exhibiting the same reactivity with both Chlamydia psittaci and Chlamydia pneumoniae in an indirect fluorescent antibody test (Immunology Letters, 23, 217-222 (1989/1990)). However, there is no description of an antigen which is recognized by the monoclonal antibody. Kuroda et al. reported a monoclonal antibody against Chlamydia pecorum (Am. J. Vet. Res, 54(5), 709-712 (1993)).

In general immunization using an elementary body or MOMP of Chlamydia pneumoniae, it is difficult to obtain cells capable of producing a monoclonal antibody which specifically reacts with an antigen site having low antigenicity in MOMP. Neither monoclonal antibodies which are specifically reactive to an antigen site having low antigenicity in MOMP nor cells producing such antibodies are available at present.

Iijima et al. reported a monoclonal antibody against a 53 KDa protein as a monoclonal antibody against Chlamydia pneumoniae (Y. Iijima, J. Clinical Microbiology, 32(3), 583-588 (1994)).

Cho-chou Kuo et al. reported that an inclusion body of Chlamydia pneumoniae can be stained by an indirect fluorescent antibody technique using a monoclonal antibody (RR402) specific to Chlamydia pneumoniae (J. Clinical Microbiology, 24(6), 1034-1037 (1986) and Japanese Unexamined Patent Publication No. Sho 64-500083). However, there is no description of an antigen which is recognized by the monoclonal antibody.

Izutsu et al. reported a monoclonal antibody reactive with Chlamydia pneumoniae (Japanese Unexamined Patent Publication No. Hei 7-16098). They showed that an inclusion body can be stained by an indirect fluorescent antibody technique using the monoclonal antibody.

The monoclonal antibody against LPS which were reported by Harland D, Caldwell et al. has a reactivity with other species of genus Chlamydia than Chlamydia pneumoniae and it is therefore difficult to detect specifically Chlamydia pneumoniae using this monoclonal antibody.

The monoclonal antibodies against MOMP which were reported by Ellena M, Peterson et al. and Byron E. Batteiger et al. have a reactivity with other species of genus Chlamydia than Chlamydia pneumoniae and it is therefore difficult to detect specifically Chlamydia pneumoniae using these monoclonal antibodies. In addition, they were not labeled.

It is not clear whether the antibody reported by Iijima et al. can detect specifically Chlamydia pneumoniae. Futhermore, there is no disclosure that an inclusion body of Chlamydia pneumoniae can be stained by using the monoclonal antibody.

The methods used by Cho-chou et al. and Izutsu, which are indirect fluorescent antibody techniques, need a long time to perform antigen tests and they are therefore unsuitable for treating a large number of samples in a short time in clinical examinations.

An object of the invention is to provide monoclonal antibodies having a reactivity specific to a Chlamydia pneumoniae-derived MOMP. The monoclonal antibodies can be used to detect specifically Chlamydia pneumoniae and to treat a large number of samples in a short time in clinical examinations.

The subject matters of the invention are as follows:
(1) A monoclonal antibody having a reactivity specific to a Chlamydia pneumoniae-derived major outer membrane protein.
(2) The monoclonal antibody of (1), which has no reactivity with a Chlamydia trachomatis-derived major outer membrane protein.
(3) The monoclonal antibody of (1), which has a reactivity with an elementary body of Chlamydia pneumoniae.
(4) The monoclonal antibody of (3), which has no reactivity with a Chlamydia trachomatis-derived major outer membrane protein.
(5) The monoclonal antibody of (1), wherein the antibody is labeled.
(6) The monoclonal antibody of (1), wherein the antibody is produced by hybridoma cell line CP-6 (FERM BP-5155) or CP-11 (FERM BP-5156).
(7) The monoclonal antibody of (6), wherein the antibody is produced by hybridoma cell line CP-6 (FERM BP-5155) or CP-11 (FERM BP-5156) and subsequently labeled.
(8) A monoclonal antibody having a reactivity with a 53 KDa antigenic protein of Chlamydia pneumoniae and being labeled.
(9) The monoclonal antibody of (8), which has no reactivity with an inclusion body of Chlamydia trachomatis and Chlamydia psittaci.
(10) The monoclonal antibody of (8), wherein the antibody is produced by hybridoma cell line AY6E2E8 (FERM BP-5154) and subsequently labeled.
(11) A method for producing an antibody producing cell that produces a monoclonal antibody having a reactivity specific to a protein, which comprises subjecting an animal to immunization with one of a native and a denatured protein and to a subsequent booster with the other protein, obtaining an antibody producing cell and fusing the antibody producing cell with a myeloma cell.
(12) The method of (11), which comprises subjecting an animal to immunization with a native protein and to a subsequent booster with a denatured protein, obtaining an antibody producing cell and fusing the antibody producing cell with a myeloma cell.
(13) The method of (11), wherein the denatured protein is obtained by treating a native protein with a detergent or a reductant.
(14) The method of (11), wherein the protein is a major outer membrane protein of Chlamydia.
(15) A monoclonal antibody producing cell which is produced by the method of (11).
(16) A monoclonal antibody producing cell capable of producing the monoclonal antibody of (1).
(17) The monoclonal antibody producing cell of (16), which is hybridoma cell line CP-6 (FERM BP-5155) or CP-11 (FERM BP-5156).
(18) A method for producing a monoclonal antibody, which comprises culturing the monoclonal antibody producing cell of (15).
(19) A method for producing a monoclonal antibody, which comprises culturing the monoclonal antibody producing cell of (16).
(20) A method for detecting and/or measuring Chlamydia pneumoniae by using the monoclonal antibody of (1).
(21) The method of (20), wherein the monoclonal antibody is produced by hybridoma cell line CP-6 (FERM BP-5155) or CP-11 (FERM BP-5156) and subsequently labeled.
(22) A method for detecting and/or measuring Chlamydia pneumoniae by using the monoclonal antibody of (8).
(23) The method of (22), wherein the monoclonal antibody is produced by hybridoma cell line AY6E2E8 (FERM BP-5154) and subsequently labeled.
(24) A reagent for detecting and/or measuring Chlamydia pneumoniae, which comprises the monoclonal antibody of (1).
(25) The reagent of (24), wherein the monoclonal antibody is produced by hybridoma cell line CP-6 (FERM BP-5155) or CP-11 (FERM BP-5156) and subsequently labeled.
(26) A reagent for detecting and/or measuring Chlamydia pneumoniae, which comprises the monoclonal antibody of (8).
(27) The reagent of (26), wherein the monoclonal antibody is produced by hybridoma cell line AY6E2E8 (FERM BP-5154) and subsequently labeled.
(28) A method for diagnosing a Chlamydia pneumoniae infection by using the monoclonal antibody of (1).
(29) The method of (28), wherein the monoclonal antibody is produced by hybridoma cell line CP-6 (FERM BP-5155) or CP-11 (FERM BP-5156) and subsequently labeled.
(30) A method for diagnosing a Chlamydia pneumoniae infection by using the monoclonal antibody of (8).
(31) The method of (30), wherein the monoclonal antibody is produced by hybridoma cell line AY6E2E8 (FERM BP-5154) and subsequently labeled.
(32) A diagnostic agent for a Chlamydia pneumoniae infection, which comprises the monoclonal antibody of (1) as an active ingredient.
(33) The diagnostic agent of (32), wherein the monoclonal antibody is produced by hybridoma cell line CP-6 (FERM BP-5155) or CP-11 (FERM BP-5156) and subsequently labeled.
(34) A diagnostic agent for a Chlamydia pneumoniae infection, which comprises the monoclonal antibody of (8) as an active ingredient.
(35) The diagnostic agent of (34), wherein the monoclonal antibody is produced by hybridoma cell line AY6E2E8 (FERM BP-5154) and subsequently labeled.

Preferred monoclonal antibodies having a reactivity specific to a Chlamydia pneumoniae-derived MOMP include monoclonal antibodies that have a reactivity specific to a Chlamydia pneumoniae-derived MOMP and which have no reactivity with Chlamydia trachomatis-derived MOMP and monoclonal antibodies that have a reactivity specific to a Chlamydia pneumoniae-derived MOMP and which have a reactivity with an elementary body of Chlamydia pneumoniae. More preferred are monoclonal antibodies that have a reactivity specific to a Chlamydia pneumoniae-derived MOMP and which have no reactivity with Chlamydia trachomatis-derived MOMP and which further have a reactivity with an elementary body of Chlamydia pneumoniae.

Specific examples of the monoclonal antibodies that have a reactivity specific to a Chlamydia pneumoniae-derived MOMP include a monoclonal antibody produced by hybridoma cell line CP-6, one that is produced by hybridoma cell line CP-11 and the like. Hybridoma cell lines CP-6 and CP-11 have been deposited with the National Institute of Bioscience and Human-Technology, the Agency of Industrial Science and Technology (1-3, Higashi 1 chome Tsukuba-shi Ibaraki-ken 305, Japan) as FERM BP-5155 and FERM BP-5156 under the terms of the Budapest Treaty.

Preferred monoclonal antibodies having a reactivity with a 53 KDa antigenic protein of Chlamydia pneumoniae are those which have no reactivity with inclusion bodies of Chlamydia trachomatis and Chlamydia psittaci.

Specific examples of the monoclonal antibodies having a reactivity with a 53 KDa antigenic protein of Chlamydia pneumoniae include a monoclonal antibody produced by hybridoma cell line AY6E2E8 and the like. Hybridoma cell line AY6E2E8 has been deposited with the National Institute of Bioscience and Human-Technology, the Agency of Industrial Science and Technology as FERM BP-5154 under the terms of the Budapest Treaty.

The invention also relates to a method for producing an antibody producing cell that produces a monoclonal antibody having a reactivity specific to a protein, which comprises subjecting an animal to immunization with one of a native protein and a denatured protein and to a subsequent booster with the other protein, obtaining an antibody producing cell and fusing the antibody producing cell with a myeloma cell.

The method of the invention for producing an antibody producing cell is characterized by the immunization of an animal except that it can be accomplished by general steps comprising immunization of an animal, cell fusion, selection of a fused cell, selection of a specific antibody producing cell, cloning and the like.

The proteins used in the method of the invention include MOMP of genus Chlamydia, particularly Chlamydia pneumoniae, bacteria, viruses, various proteins derived from animals including human and plants, and the like.

Native proteins are defined as proteins whose original steric structures are maintained and include Chlamydia, bacterial, viral organisms per se, for example, EB of Chlamydia, as well as partial and complete purification products of proteins. The native proteins are used as antigens for immunization. Antigens derived from various strains of Chlamydia pneumoniae can be used as native proteinaceous antigens. For example, EBs of strains YK-41 and TWAR can be used.

Denatured proteins are defined as proteins in such a condition that their original steric structures formed in a physiological solution have been destroyed by a certain treatment and proteins in such a condition that their disulfide bonds (S-S bonds) have also been cleaved and their amino acid sequences are recognized as antigen sites are preferred. The denatured proteins are also used as antigens for immunization. Since a cell producing an antibody against a site having low antigenicity in a denatured protein is sufficiently amplified by the immunization with the denatured protein, the antibody producing cells can be easily obtained even in the case where a cell producing a monoclonal antibody against a native protein is difficult to obtain if immunization with the native protein alone is performed.

In the case where either the native protein or the denatured protein is used as an antigen for immunization, only the formation of antibody against a site having high antigenicity in the protein is induced and it is difficult to induce efficiently the formation of an antibody against a desired site having low antigenicity.

MOMP and EB of Chlamydia pneumoniae to be used as antigens for immunization can be obtained, for example, from strain YK-41 by the method described in the following examples.

A method for denaturing the proteins include but are not limited to methods of treatment with a reductant or a detergent, a method of heat treatment, methods of treatment with an organic solvent and the like. The methods of treatment with a reductant or a detergent are preferred. Reductants include but are not limited to 2-mercaptoethanol. Detergents include but are not limited to SDS (sodium dodecybenzene-sulfonate).

Mammals to be immunized include mouse, rat, rabbit, sheep, chicken and the like. Mouse is especially preferred.

The mammal is subjected to immunization with one of the native protein and the denatured protein and to a final immunization with the other protein, preferably to immunization with the native protein and to a final immunization with the denatured protein. In the case of Chlamydia pneumoniae, it is recommendable to immunize the mammal with EB or native MOMP of Chlamydia pneumoniae and finally with the denatured protein, for example, MOMP which has been denatured by treatment with a reductant or a detergent.

Furthermore, it is preferred to use an adjuvant in the immunization. Preferred examples of the adjuvant include Freund's complete adjuvant (hereinafter abbreviated to "FCA"), Freund's incomplete adjuvant (hereinafter abbreviated to "FIA") and the like.

The immunization can be performed generally by 1-5 or more injections of the antigen. The amount of the antigen to be injected may range generally from 1 ng to 10 mg per mammal and can be varied depending on the antigen.

The final immunization can be performed generally by 1-5 or more injections of the antigen. The amount of the antigen to be injected may range generally from 1 ng to 10 mg per mammal and can be varied depending on the antigen. In general, the amount of the antigen to be used in final immunization is 1/100 to 1/10 of the amount used in the immunization.

After sufficient immunization by the above method, spleen cells, lymphocyte B cells or the like are separated from the immunized mammal so that they are used as parent cells capable of producing an antibody.

As the other parent cell for the preparation of a hybridoma, myeloma cells can generally be used. For example, mouse-derived P3U1, NS-1, 653, SP2, X63, MPC-11 and the like, rat-derived AG1, AG2, AG3, RCY3, 210 and the like, human-derived SKO-007 and the like can be used. Mouse-derived cells are preferred and P3U1, 653 and the like are particularly preferred.

Cell fusion can be performed by a method using polyethylene glycol, a method using Sendai virus, electrofusion and the like. A method using polyethylene glycol is preferred.

Hybridomas can be selected by culture in selection media where only the interested hybridomas can be grown. For example, HAT (hypoxanthine aminopterin thymidine) medium, HAz (hypoxanthine azaserine) medium and the like are preferred media.

Specific antigen producing cells can be selected by recovering supernatants of wells in which the desired hybridoma has grown in the culture and checking for the presence or absence of antibody formation by enzyme antibody techniques or immuno blot techniques using a desired protein (e.g., EB or MOMP of Chlamydia pneumoniae) and the like.

Cloning is defined as a procedure by which the specific antibody producing cells are obtained as a homogeneous cell population derived from one clone. Cloning can be performed by a limiting dilution-cultured method, a method of picking out a colony on a soft agar, a single cell manipulation method, a FACS method or the like. A limiting dilution-cultured method is simple and therefore preferred.

The monoclonal antibody producing cells can be obtained by the aforementioned methods.

The monoclonal antibody producing cells obtained by the methods of the invention include hybridoma cell lines CP-6 and CP-11.

The monoclonal antibody producing cells obtained by the aforementioned methods may be cultured in an appropriate medium to produce the monoclonal antibodies. Alternatively, the monoclonal antibodies can be produced by injecting the obtained hybridomas in a mouse or the like and collecting ascites.

Culture media include a modified Eagle's medium supplemented with fetal calf serum (MEM medium), a Dulbecco's modified Eagle's medium (DMEM medium), RPM11640 medium and the like.

The monoclonal antibodies can be recovered from culture supernatants by affinity chromatography using a protein A column, protein G column or the like, ion exchange chromatography, polyethylene glycol fractionation, ethanol fractionation, ammonium sulfate fractionation and the like. Affinity chromatography is preferred, with the use of a protein A column being more preferred.

The obtained monoclonal antibodies can be examined for a reactivity specific to a desired protein. For example, a monoclonal antibody which specifically reacts with a desired protein can be selected by a Western blot method using an organism which is treated with an appropriate solution and electrophoresed. More specifically, the monoclonal antibodies can be examined for a reactivity specific to MOMP or EB of Chlamydia pneumoniae, and/or for a reactivity with a 53 KDa antigenic protein. The reactivity specific to MOMP of Chlamydia pneumoniae can be evaluated by detecting for the presence of an antibody which specifically reacts with a band of MOMP having a molecular weight of about 39.5 KDa by a Western blot method using EB of Chlamydia pneumoniae which is treated with an appropriate solution and electrophoresed. The monoclonal antibodies which are evaluated to have a reactivity specific to MOMP of Chlamydia pneumoniae are believed to recognize an amino acid sequence of MOMP of Chlamydia pneumoniae as an epitope.

The antigenic protein which is reactive with the monoclonal antibody can be studied by preparing a cDNA library of Chlamydia pneumoniae using λ gt11 and the like, expressing the cDNA to produce peptides, reacting the monoclonal antibody with the produced peptides and analyzing the codons of cDNA that specifically reacts with the monoclonal antibody and which is inserted into the λ phage.

Any types (globulin classes) of antibodies can be produced by the method of the invention for producing a monoclonal antibody. Fragments of the monoclonal antibodies and molecules having a fragment of the monoclonal antibody can be used.

Monoclonal antibodies produced by the method of the invention can recognize as an epitope a site having low antigenicity in a protein which has heretofore been difficult to obtain. More specifically, they can recognize the planar structure or amino acid sequence of a protein as an epitope.

The monoclonal antibodies having a reactivity specific to Chlamydia pneumoniae-derived MOMP and the monoclonal antibodies having a reactivity with a 53 KDa antigenic protein of Chlamydia pneumoniae can be obtained, for example, by culturing the monoclonal antibody producing cell (hybridoma) produced by the method of the invention and collecting culture supernatants, followed by purification as described above. The immunization used in the method for producing a monoclonal antibody producing cell may be performed by a known technique.

Preferably, the obtained monoclonal antibodies of the invention are not substantially reactive with other species of genus Chlamydia, i.e., Chlamydia trachomatis and Chlamydia psittaci. In particular, monoclonal antibodies which are not substantially reactive with MOMP of all other species of genus Chlamydia than Chlamydia pneumoniae and monoclonal antibodies which are specifically reactive with a 53 KDa antigenic protein of Chlamydia pneumoniae are more preferred because they are useful in diagnosis of diseases which are species-specific to Chlamydia pneumoniae.

If the monoclonal antibodies have a reactivity with species other than Chlamydia pneumoniae in a strict sense but exhibit only low reactivity in a direct or indirect fluorescent antibody technique, they can be distinguished from those exhibiting high reactivity and therefore can practically be used in diagnosis. Such monoclonal antibodies are also useful. It is important that the monoclonal antibodies should not have cross-reactivity with Chlamydia trachomatis which is frequently separated but it is not so important whether they have cross-reactivity with Chlamydia pecorum and Chlamydia psittaci which are less frequently separated. If the monoclonal antibodies react highly with Chlamydia pneumoniae by a direct or indirect fluorescent antibody technique but react only weakly with another species (e.g., Chlamydia psittaci) while reacting not at all with the other species (e.g., Chlamydia trachomatis and Chlamydia pecorum), they can be used to determine which species of Chlamydia causes a disease and are therefore preferred.

The monoclonal antibodies of the invention include any types (globulin classes) of antibodies. Fragments (Fab, Fab', Fab'₂ and the like) of the monoclonal antibodies and molecules having a fragment of the monoclonal antibody are also included.

The monoclonal antibodies having a reactivity specific to Chlamydia pneumoniae-derived MOMP and the monoclonal antibodies having a reactivity with a 53 KDa antigenic protein of Chlamydia pneumoniae may be labeled.

The monoclonal antibodies can be labeled by binding a labeling agent such as various kinds of dyes, colloids, enzymes and the like to the monoclonal antibodies.

The dyes include fluorescent dyes such as FITC (Fluorescein 5-isothiocyanate), rhodamine, fluoresceine and the like. FITC is preferred because of its general applicability and easy availability. FITC can be purchased from Sigma Co., Ltd.

The colloids include a gold colloid and the like.

The enzymes include peroxidase, alkaline phosphatase, luciferase, β-galactosidase and the like.

The labeling agent can be bound to the monoclonal antibodies by any known techniques such as the methods described in "EXPERIMENTAL IMMUNOLOGY", edited by Ivan Lefkovits et al., Nishimura Shoten, (1985).

In addition, a chemical substance such as biotin, avidin, streptoavidin, digoxygenin and the like may be inserted between the monoclonal antibody and the labeling agent.

Since FITC is bound to a lysine residue in antibodies, some antibodies may lose their antigen-binding activity as the result of labeling with FITC.

Chlamydia pneumoniae can be detected and/or measured, for example, by using the monoclonal antibody of the invention after it is directly or indirectly labeled. The monoclonal antibodies of the invention can be labeled directly by the method described above. The monoclonal antibodies of the invention can be labeled indirectly by using an antigen that can recognize and bind to the monoclonal antibody and which is labeled with a labeling agent (labeled secondary antibody). The secondary antibody includes a rabbit anti-mouse immunoglobulin antibody and the like. In the case where the labeling agent is a fluorescent dye, it is irradiated with ultraviolet light and the emitted fluorescence is detected and/or measured. In the case where the labeling agent is an enzyme, a substrate is added and luminescence or color development which occurs by the catalytic action of the enzyme is detected and/or measured. In both cases, a sensitizer may be used, if necessary.

The reagents for detecting and/or measuring Chlamydia pneumoniae include the unlabeled or labeled monoclonal antibodies of the invention per se, and combinations thereof with at least one of the following components.
(1) substances for suppressing non-specific immunoreactions (e.g., bovine serum albumin)
(2) substrates for the enzymes used as labelling agents
(3) sensitizers
(4) control antigens (MOMP, 53 KDa antigenic protein of Chlamydia pneumoniae)
(5) labeled secondary antibodies

Preferably, these components, taken either individually or in admixtures of two or more, are lyophilized to make preparations that are convenient to use in clinical practice.

The reagents for detecting and/or measuring Chlamydia pneumoniae can directly be used as agents for diagnosing Chlamydia pneumoniae infections.

The monoclonal antibodies of the invention can be used for the diagnosis of Chlamydia pneumoniae infections. Chlamydia pneumoniae infections can be diagnosed by using the reagents for detecting and/or measuring Chlamydia pneumoniae or the agents for diagnosing Chlamydia pneumoniae infections. When a sample exhibits a positive signal in a test using one of these reagents and diagnostic agents, the contraction of Chlamydia pneumoniae infection is verified.

It is preferred to use a combination of the monoclonal antibodies of the invention which recognize different antigens because the amount of the antigen varies with samples.

Examples of the monoclonal antibodies that may be combined include the monoclonal antibody having a reactivity with MOMP of Chlamydia pneumoniae and the monoclonal antibody having a reactivity with a 53 KDa antigenic protein of Chlamydia pneumoniae.

In addition, the monoclonal antibodies of the invention may be combined with other monoclonal antibodies or polyclonal antibodies.

The monoclonal antibodies of the invention and those produced by the method of the invention can be used in methods for detecting a desired protein, specifically methods using immunoreactions such as radioimmunoassay, enzymeimmunoassay and the like. They can also be used as a component of diagnostic agents or reagents for research study. If desired, they can be used in pharmaceutical preparations either directly or as chimeric or drafted antibodies.

Additionally, the monoclonal antibodies of the invention and those produced by the method of the invention can be used in kits for diagnosis in which they are combined with a polymerase chain reaction.

The antibody producing cells of the invention can also be used as sources of antibody genes. The antibody genes can be expressed in various kinds of cells.

The above disclosure generally describes the invention. A more complete understanding can be obtained by reference to the following specific examples which are provided herein for the purpose of illustration only, and are not intended to limit the scope of the invention.

### EXAMPLE 1

### Preparation of a Monoclonal Antibody

### Specific to the MOMP of C. pneumoniae (1)

### 1.1 preparation of the EB of C. pneumoniae

For the preparation of the EB of *C*. *pneumoniae*, strain YK-41 was used. The infection of human lung cells (hereinafter referred to as "HL cells") with strain YK-41 was carried out in accordance with the method of Kishimoto et al. [KENSA TO GIJUTU (Test and Technique) 18(7), 959-964 (1990)]. The infected cells which were cultured in a 24- or 6-well petri dish were scratched and recovered together with the medium, followed by centrifugation at 6,000 rpm for 30 minutes. After the removal of the supernatant, the precipitate was suspended in a mixed solution of sucrose, phosphate and glutamic acid [containing 7.5% (w/v) sucrose, 3.8 mM KH₂PO₄, 7 mM K₂HPO₄ and 5 mM glutamic acid, (pH 7.4) hereinafter referred to as "SPG"]. The suspension was disrupted with a homogenizer and then centrifuged at 2,500 rpm for 10 minutes. The supernatant was removed and the precipitate was suspended in SPG. These procedures were repeated 5 times and then the supernatant was recovered. The recovered supernatant was mixed with 23% (v/v) Urografin (Nihon Schering K.K.) and 50% (w/v) sucrose at a volume ratio of supernatant:Urografin:sucrose = 2:2:1, followed by centrifugation at 8,000 rpm for 1 hour. After the removal of the supernatant, the lower layer [50% (w/v) sucrose layer] was recovered, suspended in SPG, washed and centrifuged at 10,000 rpm for 30 minutes. After the removal of the supernatant by suction, the precipitate was suspended in SPG, mixed with 26.6 - 38% (v/v) Urografin density gradients at a volume ratio of 1:4 and centrifuged at 8,000 rpm for 1 hour. The resultant intermediate layer was recovered and suspended in SPG. After centrifugation at 10,000 rpm for 30 minutes, the supernatant was removed by suction and the precipitate was suspended in an appropriate amount of SPG, to thereby prepare an EB solution. After dispensation, this solution was stored at 4°C or -70°C.

### 1.2 Preparation of the MOMP of C. pneumoniae

The EB solution obtained in 1.1 above was separated and purified by disc preparative electrophoresis to thereby prepare the MOMP of *C*. *pneumoniae*. In brief, 0.5 ml of the obtained EB solution [protein (EB) concentration: 727 µg/ml] was mixed with 0.5 ml of 2X sample buffer [0.31 M Tris-HCl (pH 6.8), 1.6% (w/v) sodium dodecylsulfate (hereinafter abbreviated as "SDS"), 1 mM dithiothreitol, 16% (v/v) glycerol, 0.005% (w/v) Bromophenol Blue] and boiled at 100 °C for 3 minutes to solubilize the protein. Then, the total volume was added to disc gels (stacking gel: 4%; separating gel 10%), subjected to electrophoresis [apparatus: Disc Preparative Electrophoresis Apparatus NA-1800 manufactured by Nippon Eido K.K.; running condition: 100 V (constant voltage)] and recovered in certain volumes (0.26 ml/fraction). For each fraction, SDS slab electrophoresis was carried out (apparatus: DNA-PAGE Electrophoresis Apparatus NB-5000 manufactured by Nippon Eido K.K.; running condition 100 V (constant voltage)]. As a result, it was confirmed that the MOMP with a molecular weight of 39.5 K was recovered in the 33rd to 45th fractions (protein concentration: 0.21 mg/ml).

### 1.3 Preparation of Hybridomas and Monoclonal Antibodies

A physiological saline solution (0.5 ml) of the EB obtained by the above-mentioned procedures was mixed with 0.6 ml of FCA (Difco). The resultant mixture was treated with a sonicator (Branson) to thereby prepare a water-in-oil emulsion, which was used as an antigen for immunization. Five BALB/c female mice 8 weeks old were each administered intraperitoneally with 200 µl of this antigen (the amount of EB per dose: 10 µg/mouse). Six days and 12 days after the administration, immunization was carried out using FIA (Difco). Six days after this immunization, the animals were bled. For each of the sera obtained, the antibody titer was measured by dot immuno binding assay (hereinafter abbreviated as "DIBA"). The animal which exhibited the highest antibody titer was administered from the tail vein with 200 µl of the purified MOMP obtained by the above-mentioned procedures (the amount of MOMP per dose: 1 µg/mouse) for three days of final immunization from the day of the antibody titer measurement. On the following day after the completion of the 3-day final immunization, each mouse was sacrificed by cervical dislocation and its spleen was removed. Then, single cell dispersion was carried out to thereby prepare splenic cells to be used for cell fusion. The partner cell for cell fusion was P3U1 mouse myeloma cells which had been cultured in advance on Dulbecco's MEM medium supplemented with 10% (v/v) fetal bovine serum (hereinafter referred to as "10F medium") in a CO₂ incubator (Napco) at 37 °C under 5% CO₂ and saturated humidity.

The above-mentioned splenic cells (4.8 x 10⁷ ) and P3U1 mouse myeloma cells (1.2 x 10⁷ ) were mixed and centrifuged at 1,000 rpm for 10 minutes. Then, the supernatant was removed by suction. After the cell pellet was loosened by applying gentle vibrations, 0.2 ml of a cell fusion accelerator [50% (w/v) polyethylene glycol; molecular weight: 4000; manufactured by Merck). After 1 minute and 45 seconds, 10 ml of the culture solution was added over 1 minute. Further, 20 ml of the culture solution was added to thereby suspend the cells. Then, the suspension was centrifuged and separated stepwise (at 300 rpm for 3 minutes, at 500 rpm for 3 minutes and at 700 rpm for 3 minutes). After the removal of the supernatant by suction, 10F medium containing 5% Briclone (Dainippon Pharmaceutical Co., Ltd.) (hereinafter referred to as "10F + Bri medium") was added to the cell pellet to thereby prepare a cell suspension. The cell suspension was dispensed into a 96-well flat-bottom multiplate (Corning) to give a cell density of 4 x 10⁴ myeloma cells/well/0.1 ml, and then incubated at 37 °C under 5% CO₂ and saturated humidity.

On the following day, HAT-containing 10F medium (Dulbecco's MEM medium supplemented with 1 x 10⁻⁴ M hypoxantine, 4 x 10⁻⁷ M aminopterin, 1.6 x 10⁻⁵ M thymidine and 10% (v/v) fetal bovine serum; hereinafter referred to as "HAT medium") was added to the wells in a volume of 0.1 ml/well. Further, a half of the culture supernatant was replaced with HAT medium every 3 to 4 days; thus, so-called HAT selection was carried out for about 2 weeks. The supernatants of those wells in which the growth of a hybridoma was observed were collected and screened by DIBA for the production of an antibody against the EB.

The DIBA was performed as follows using a 96-well U-bottom multiplate (Corning). To a 3 mm square membrane filter (Advantec; a 47 mm diameter latticed filter; Cat. No. A045b047A) on which the EB had been immobilized as an antigen in an amount of 0.02 µg/dot in advance, 10% (v/v) FCS (fetal calf serum) was added and reacted at room temperature for 15 minutes. Then, after the removal of the supernatant, 100 µl of the culture supernatant of the hybridoma was added and reacted at room temperature for 1 hour. Then, after the removal of the supernatant, 100 µl of a 500-fold dilution of peroxidase-labeled rabbit anti-mouse immunoglobulin antibody (Cappel Inc.) was added and reacted at room temperature for 1 hour. Finally, 4-chloro-1-naphthol was added as a substrate to thereby color the reaction solution. Those wells in which coloring was observed with the naked eye on the antigen-immobilized membrane filter were judged positive in antibody production.

The hybridomas in those wells which were found positive in antibody production as the result of the above screening were subcloned by the limiting dilution method. In brief, the antibody production positive hybridoma was suspended in HAT medium and the cell density of the suspension was measured. Then, the suspension was diluted, dispensed into a new microplate to give a cell density of from 2 to 100 cells/100 µl/well and then incubated. As the diluent, HT + 5% Briclone medium was used.

When the growth of a hybridoma was observed in a well with a high dilution, the hybridoma was determined for the ability to produce an antibody and observed microscopically to confirm that it was a monoclone. Then, the same procedures were repeated to thereby yield anti-EB monoclonal antibody producing clones which had a high capability for sustained antibody production. For each of the clones thus obtained, the culture scale was gradually increased. Finally each clone was cultured in 50 ml of 10F medium and the culture supernatant was examined for the characteristics of the antibody produced.

The experimental procedures so far mentioned were carried out twice and finally there were obtained 10 strains of the monoclonal antibody producing clones shown in Table 1.

The globulin class of the monoclonal antibodies produced by the above-mentioned 10 hybridoma clones were determined using ISOTYPE Ab-STAT-1 kit (Sang Stat Medical) (see Table 1).

As shown in Table 1, monoclonal antibodies CP-1, CP-2, CP-4, CP-5, CP-6, CP-7 and CP-8 did not exhibit reactivity to *C*. *trachomatis* but exhibited reactivity to *C*. *pneumoniae*.

Next, the proliferated cells obtained by culturing each of the hybridoma clones producing the monoclonal antibodies shown in Table 1 in 10F medium were inoculated in an amount of 1 x 10⁷ cells/mouse into the abdominal cavities of BALB/c mice which had been pretreated with 0.5 ml of Pristan (Wako Purechemical Co., Ltd.) per mouse. Two to three weeks after the inoculation, ascites was obtained from each mouse and the monoclonal antibodies of the present invention were purified therefrom.

The ascites which was obtained by inoculating the hybridoma clone No. 6 was purified by the method described below where a protein A-immobilized affinity column was used. In brief, to the mouse ascites, an equal volume of a mixture of 1.5 M glycine and 3 M NaCl (pH 8.9) was added. The resultant mixture was passed through a 0.45 µm nylon filter (Corning), applied to an affinity column of Protein A-Poros (NGK Insulators Ltd.) and washed with a mixture of 1.5 M glycine and 3 M NaCl (pH 8.9). Then, the antibody was eluted with phosphate-citrate buffer (pH 6.0) containing 150 mM NaCl.

After neutralization with 1 M Tris-HCl buffer (pH 9.0), the eluate was cooled and dialyzed against PBS overnight. The recovered solution was measured for the absorbance at 280 nm (using Model U-2000, Hitachi, Ltd.), passed through a 0.2 µm cellulose acetate filter (Corning) and stored at -80°C, to thereby prepare a stock solution of purified antibody.

### 1.4 Specificity Analysis of the Monoclonal Antibodies

The specificity analysis of the monoclonal antibodies was performed by an immunoblotting. The reactivity with *C*. *pneumoniae* was examined using the EB solution (EB concentration: 727 µg/ml) obtained by the method described in 1.1 above. Specifically, the EB solution containing 50 µg of EB and a sample buffer [0.063 M Tris, 5% (v/v) 2-mercaptoethanol, 10% (v/v) glycerol, 2.3% (w/v) SDS, 0.001% (w/v) BPB (Bromphenol Blue)(pH 6.8)] were mixed at a volume ratio of 1:1 and boiled for 10 minutes. Then, 100 µl of the resultant mixture was subjected to electrophoresis [apparatus: AE-6560 manufactured by Atto Corporation; gel: Pagel 1020DL; electrophoresis buffer: 0.025 M Tris, 0.192 M glycine, 0.1% (w/v) SDS, (pH 8.3); running conditions: at a constant current of 20 mA, for 80 minutes]. As a transfer apparatus, NA-1510 (Nippon Eido K.K.) was used. The electrophosed gel, 2 sheets of filter paper 3 mm thick (Whatman) and a nitrocellulose membrane (Bio Rad) were soaked for 15 minutes in transfer buffer [0.025 M Tris, 0.192 M Glycine, 20% methanol (pH 8.3)] which was presoaked in ice. On a pad, there were placed the filter paper, gel, nitrocellulose membrane and filter paper in this order. Then, another pad was placed at the top and they were set in the transfer apparatus. The apparatus was placed in ice and turned on for 2 hours at a constant voltage of 50 V, to thereby transfer the protein to the nitrocellulose membrane. The nitrocellulose membrane was washed with PBS (Nissui Pharmaceutical Co., Ltd.) (for 2 minutes each time, twice, with shaking) and then blocked with 10% FCS/PBS (for 30 minutes at room temperature with shaking). Afterwards, the nitrocellulose membrane was washed with PBS (Nissui Pharmaceutical Co., Ltd.) (for 2 minutes each time, 3 times, with shaking) and reacted with each of the culture supernatants of all the hybridomas shown in Table 1 (for 1 hour at room temperature with shaking). After washing with PBS (for 2 minutes each time, 3 times, with shaking), the nitrocellulose membrane was reacted with a 500-fold dilution of peroxidase-labeled rabbit anti-mouse immunoglobulin antibody (Cappel Inc.) for 1 hour at room temperature with shaking. The nitrocellulose membrane was washed with PBS (for 2 minutes each time, 3 times, with shaking) and then a color forming solution (1 ml of 3 mg/ml 4-chloro-1-naphtol, 5 ml of PBS, 2 µl of aqueous hydrogen peroxide) was added and reacted until protein bands appeared. Then, the reaction was terminated by washing the membrane with distilled water several times.

The analysis of the reactivity with *C*. *trachomatis* was carried out using the purified EB of *C*. *trachomatis* (strain L2) by an immunoblotting under similar conditions as mentioned above.

As a result, the two monoclonal antibodies, CP-6 and CP-7, of the present invention did not exhibit reactivity with the MOMP of *C*. *trachomatis*, while exhibiting reactivity with the MOMP of *C*. *pneumoniae*.

### 1.5 Analysis of the Antibodies by the Fluorescent Antibody Technique (Reactivity with C. trachomatis, C. psittaci and C. pecorum)

An isolate from each of *C*. *trachomatis*, *C*. *psittaci* and *C*. *pecorum* was spotted (1 µl) on a slide glass and each of the culture supernatants of all the hybridomas shown in Table 1 was placed thereupon (8 µl). Then, the slide glass was placed in a humid box, where a reaction was carried out at 37°C for 3 hours. The slide glass was washed with PBS and distilled water, followed by air drying. After air drying, 8 µl of a 10-fold dilution of FITC-labeled anti-mouse immunoglobulin/goat serum (KPL Inc.) was placed on the slide glass so that all spot sites were covered. The slide glass was placed in a humid box, where a reaction was carried out at 37°C for 30 minutes. Then, the slide glass was washed and air dried. Thereafter, 100 µl of an inclusion solution (50% glycerol-PBS) was added to form inclusion bodies, which were then examined with a fluorescence microscope. As a result, the two monoclonal antibodies, CP-6 and CP-7, of the present invention could be observed as bright points only when they were with the EB of *C*. *pneumoniae*, which indicated that these antibodies were reactive with the EB.

The hybridoma which produced the above-mentioned monoclonal antibody CP-6 was deposited as hybridoma cell line CP-6 at the National Institute of Bioscience and Human-technology, Agency of Industrial Science and Technology (Accession No. FERM P-14436; BP-5155).

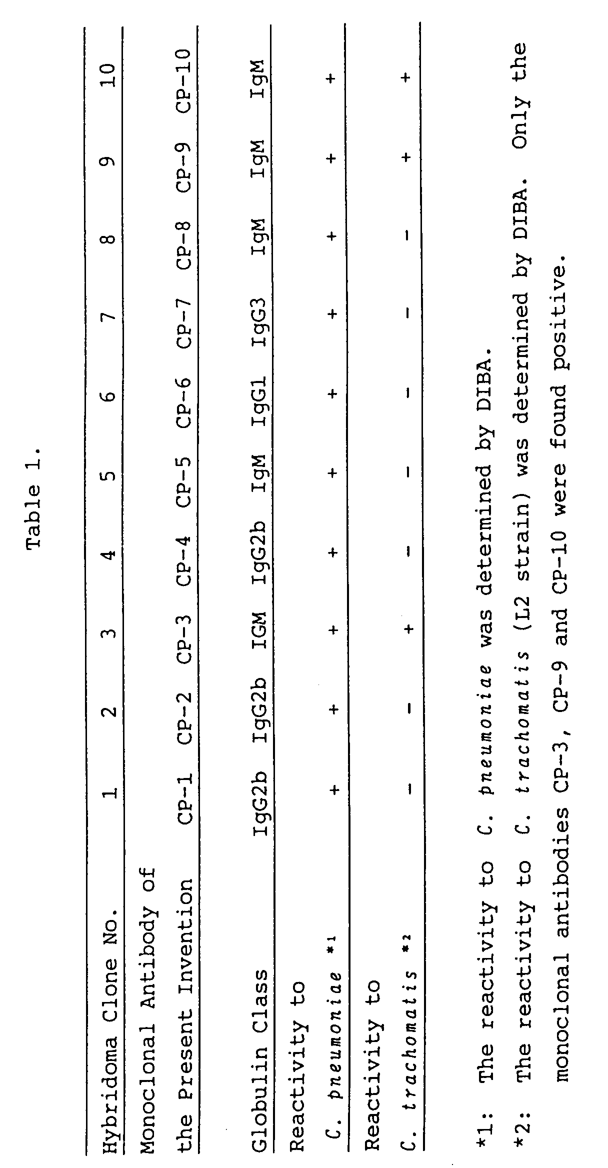

### EXAMPLE 2

### Preparation of A Monoclonal Antibody

### Specific to the MOMP of C. pneumoniae (2)

One strain of monoclonal antibody-producing clone (CP-11) was obtained in substantially the same manner as described in 1.1 to 1.3 in Example 1 and a stock solution of the antibody CP-11 was prepared.

The globulin class of the monoclonal antibody produced by the hybridoma clone CP-11 was found IgG1 as a result of the measurement using ISOTYPE Ab-STAT-1 Kit (Sang Stat Medical).

The specificity of the monoclonal antibody produced by the hybridoma clone CP-11 was analyzed in substantially the same manner as described in 1.4 in Example 1. This monoclonal antibody did not exhibit reactivity to the MOMP of *C*. *trachomatis*, exhibited very strong reactivity to the MOMP of *C*. *pneumoniae* and exhibited only very weak reactivity to the MOMP of *C*. *psittaci*.

Further, this monoclonal antibody was analyzed by the indirect fluorescent antibody technique in substantially the same manner as described in 1.5 in Example 1. The results are shown in Table 2.

**Table 2**

| | Reactivity * |
|---|---|
| *C*. *pneumoniae* | +++ |
| *C*. *psittaci* | ± |
| *C*. *trachomatis* | - |
| *C*. *pecorum* | - |

| | |
|---|---|
| * Very strong reactivity: +++ Strong reactivity: ++ Weak reactivity: + Very weak reactivity: ± No reactivity observed: - | |

As the data show, according to the indirect fluorescent antibody technique using this monoclonal antibody, the strong reaction can be judged to indicate *C*. *pneumoniae*; the very weak reaction can be judged to indicate *C*. *psittaci*; and the absence of reaction can be judged to indicate *C*. *trachomatis* or *C*. *pecorum*.

The hybridoma which produces this monoclonal antibody was deposited as hybridoma cell line CP-11 at the National Institute of Bioscience and Human-technology, Agency of Industrial Science and Technology (Accession No. FERM P-14592; BP-5156).

From the above results, it can be judged that this monoclonal antibody recognizes the amino acid sequence of the portion of *C*. *pneumoniae* MOMP which is exposed outside of the membrane of EB.

### EXAMPLE 3

### Preparation of the Monoclonal Antibody

### Produced by Hybridoma Cell Line AY6E2E8

As a monoclonal antibody, the monoclonal antibody produced by hybridoma cell line AY6E2E8 was used. Hybridoma cell line AY6E2E8 was deposited at the National Institute of Bioscience and Human-technology, Agency of Industrial Science and Technology (Accession No. FERM P-13688; BP-5154).

To one volume of monoclonal antibody-containing ascites obtained by intraperitoneal injection of hybridoma cell line AY6E2E8 to a mouse, 3 volumes of PBS were added, mixed and centrifuged at 3,000 rpm for 10 minutes. The supernatant was filtered with a filter 0.22 µm in pore size and then purified by HPLC using Chromatop Superprotein A Column (4.6 mm dia. x 100 mm, NGK Insulators, Ltd.) which had been equilibrated with PBS in advance.

The sample (1 ml) which had been filtered with the 0.22 µm filter was poured into the column, which was then washed by feeding PBS at a rate of 1 ml/min for 3 minutes and at a rate of 5 ml/min for 4 minutes. To this column, 1 liter of purified water having 8.77 g of NaCl, 16.7 g of citric acid (monohydrate) and 14.72 g of Na₂HPO₄ · 12H₂O dissolved therein was fed at a rate of 2 ml/min for 5 minutes to thereby elute the monoclonal antibody. The fractions containing the monoclonal antibody were collected. To these fractions, an aqueous solution of 2 M Tris (hydroxymethyl) aminomethane was added to adjust the pH to 7 - 9. Then, the resultant solution was stored at 4°C.

### EXAMPLE 4

### Analysis of the Antigen Protein which Reacts with the Monoclonal Antibody produced by Hybridoma Cell Line AY6E2E8

### 4.1 Preparation of the Genomic DNA of C. pneumoniae Strain YK-41

A suspension (300 µl) of the purified EB of *C*. *pneumoniae* strain YK-41 obtained in 1.3 above (protein concentration: 1.37 mg/ml) was centrifuged at 4°C for 5 minutes at 12,000 rpm. The precipitate was suspended in 500 µl of 10 mM Tris-HCl buffer (pH 8.0) containing 1 mM EDTA (hereinafter referred to as "TE buffer"). A similar centrifugation was carried out again and then the precipitate was suspended in 300 µl of TE buffer. To the suspension, 30 µl of 1% SDS aqueous solution and 30 µl of 1 mg/ml aqueous proteinase K solution were added and incubated at 56 °C for 30 minutes, to thereby dissolve the EB. To the resultant solution, 350 µl of 0.1 M Tris-HCl buffer (pH 8.0) saturated phenol was added and mixed well with a vortex mixer. Then, the mixture was centrifuged at 4°C for 5 minutes at 12,000 rpm and the aqueous layer was recovered (extraction of DNA). This extraction operation was repeated again. Then, 2 µl of 10 mg/ml RNase solution was added to the recovered layer and incubated at 37 °C for 2 hours, to thereby decompose the RNA. To the resultant solution, 300 µl of a 25:24:1 (volume ratio) mixture of 0.1 M Tris-HCl buffer (pH 8.0) saturated phenol, chloroform and isoamyl alcholol (hereinafter referred to as "PCI") was added, mixed well with a vortex mixer and centrifuged at 4°C for 5 minutes at 12,000 rpm. Then, the resultant aqueous layer was recovered. This operation was repeated 5 times in total.

To the obtained solution, 1/10 volume of 10 M aqueous ammonium acetate solution and 2 volumes of ethanol were added, left for 5 minutes to precipitate the DNA and then centrifuged at 4 °C for 5 minutes at 12,000 rpm. As to the precipitation operation, 600 µl of 70% aqueous ethanol solution was added, mixed and centrifuged at 4 °C for 5 minutes at 12,000 rpm and this procedure was repeated twice. The centrifuge tube was then left for 15 minutes with its cap open to dry the precipitate. The precipitate was dissolved in 200 µl of TE and stored at -20 °C.

### 4.2 Preparation of the Genomic DNA Expression Library

To 100 µl of the genomic DNA solution obtained, 10 µl of M-buffer for restriction enzymes and 10 µl of a restriction enzyme mixture (obtained by mixing 0.4 µl each of AccI, HaeIII and 1/50 dilution of AluI with 20 µl of TE) were added and incubated at 37 °C for 20 minutes. The reaction time 20 minutes is the duration of time required for the genomic DNA to be decomposed to partially digested DNA fragments 1 kbp - 7 kbp in size. This reaction time was tested in advance using a small amount of the genomic DNA. To the above reaction solution, 100 µl of PCI was added, mixed well with a vortex mixer and centrifuged at 4 °C for 5 minutes at 12,000 rpm, and then the aqueous layer was recovered. 3M aqueous sodium acetate solution (10 µl) and ethanol (220 µl) were added to the aqueous layer and left to stand at -80 °C for 15 minutes, to thereby precipitate the partially digested DNA. After centrifugation at 4°C for 5 minutes at 12,000 rpm, the supernatant was discarded. To the precipitate, 500 µl of 70% aqueous ethanol solution was added, mixed and then re-centrifuged at 12,000 rpm for 5 minutes. The supernatant was discarded and the precipitate was dried under reduced pressure.

The partially digested DNA obtained above was dissolved in 20 µl of purified water. To 19 µl of this solution, 14 µl of the linker described below (20 pmole/µl), 4.5 µl of 10 mM ATP, 4.5 µl of 0.2 M Tris-HCl buffer (pH 7.6) containing 50 mM MgCl₂, 50 mM dithiothreitol and 500 µg/ml bovine serum albumin (hereinafter referred to as "10X ligation buffer"), 2 µl of purified water and 1 µl of T4 ligase were added and incubated at 16°C for 4 hours, to thereby attach the linker to the DNA.
5'-AATTCGAACCCCTTCG-3'
3'-GCTTGGGGAAGCp-5'
The linker-attached partially digested DNA was applied to a Chroma Spin 6000 Column in which the mobile phase was 10 mM Tris-HCl buffer containing 0.1 M NaCl and 1 mM EDTA. The eluate was collected in two drops. A portion of each fraction was analyzed by 0.8% agarose gel electrophoresis to thereby recover fractions containing 1 kbp - 7 kbp DNA fragments. To 144 µl of the thus obtained fractions, 13 µl of purified water, 20 µl of 10 mM ATP, 20 µl of 0.5 M Tris-HCl buffer (pH 7.6) containing 0.1 M MgCl₂, 50 mM dithiothreitol, 1 mM spermidine hydrochloride and 1 mM EDTA (hereinafter referred at as "10X phosphorylation buffer") and 3 µl of T4 polynucleotide kinase were added and incubated at 37°C for 30 minutes, to thereby phosphorylate the 5' termini of the DNA fragments. PCI (200 µl) was added to the reaction solution, mixed well by shaking and centrifuged at 4°C for 5 minutes at 12,000 rpm. Then, the aqueous layer was recovered. To the recovered layer, 1 µl of 20 mg/ml aqueous glycogen solution, 20 µl of 3 M aqueous sodium acetate solution and 400 µl of ethanol were added to precipitate nucleotides. The resultant solution was centrifuged at 4°C for 10 minutes at 12,000 rpm and the supernatant was discarded. To the precipitate, 200 µl of ethanol was added and re-centrifuged. Then, the supernatant was discarded, and the precipitate was air dried and dissolved in 1 µl of purified water.

To 0.6 µl of the thus obtained solution, 1 µl of λ gt11 DNA (1 µg/µl, Stratagene) digested with EcoRI in advance, 0.5 µl of 10X ligation buffer, 0.5 µl of 10 mM ATP, 0.4 µl of T4 ligase and 2 µl of purified water were added and incubated at 4°C overnight. Then, using Gigapack II Gold Packaging Kit (Stratagene), the recombinant λ gt11 DNA obtained was packaged.

### 4.3 Cloning of the DNA coding for the Antigen Polypeptide

One platinum loop-full of *E*. *coli* strain Y1090r- was inoculated in LB medium (containing 5 g of NaCl, 10 g of polypeptone and 5 g of yeast extract in 1 liter of water) supplemented with 3 ml of 10 mM MgSO₄, 0.2% maltose and 50 µg/ml ampicillin, incubated at 37°C overnight under shaking and then centrifuged at 2,000 rpm for 10 minutes. To the precipitate (*E*. *coli*), 9 ml of 10 mM aqueous MgSO₄ solution was added and mixed. A portion (0.35 ml) of the resultant *E*. *coli* suspension was measured off. To this suspension, 0.1 - 10 µl of λ gt11 (DNA library) suspension was added and incubated at 37 °C for 20 minutes, to thereby infect the *E*. *coli* with the λ gt11. To 2.5 ml of liquid LB agar medium which was kept at 47 °C in advance, the λ gt11-infected *E*. *coli* mentioned above was added. Then, this medium was immediately scattered over LB agar medium. After the upper-layer agar medium became solid, the cells were incubated at 42°C for 3 to 4 hours. When plaques were observed, a nitrocellulose filter (⌀ 82 mm) soaked in 10 mM aqueous IPTG solution was placed upon the upper-layer agar medium and the cells were incubated further at 37°C for 12 hours. The nitrocellulose filter was marked at three asymmetric points by piercing with a syringe needle bearing black ink. Then, the nitrocellulose filter was removed from the agar medium and washed 3 times with 20 mM Tris-HCl buffer containing 150 mM NaCl and 0.1% Tween 20 (pH 7.5, hereinafter referred to as "TTBS buffer"). The agar medium was kept in an refrigerator.

The nitrocellulose filter was soaked in 150 mM NaCl-containing 20 mM Tris-HCl buffer (pH 7.5, hereinafter referred to as "TBS buffer") supplemented with 0.1% bovine serum albumin and shaked at 37°C for 1 hour to carry out a blocking reaction. Then, the nitrocellulose filter was washed with TTBS buffer twice, soaked in 5 - 10 µg/ml *C*. *pneumoniae*-specific monoclonal antibody (AY6E2E8) solution in TTBS and shaked at 37°C for 1 hour. The nitrocellulose filter was washed with TTBS buffer 3 times and then shaked at 37 °C for 1 hour in peroxidase-labeled (50 ng/ml) anti-mouse IgG antibody solution in TTBS buffer. The nitrocellulose filter was washed with TTBS buffer 3 times and with TBS buffer 3 times. Then, the filter was soaked in a color forming substrate solution (prepared by adding 60 µl of 30% aqueous hydrogen peroxide solution and 20 ml of 0.3% 4-chloro-1-naphtol solution in methanol to 100 ml of TBS buffer) and left at room temperature for about 30 minutes. When sufficient coloring was obtained, the filter was recovered from the solution, washed with purified water and air dried.

The plaques on the agar medium which corresponded to the colored spots on the nitrocellulose filter were sought and identified. The agar at these portions was pierced with a Pasteur's pipette to recover the plaques. The recovered plaques were placed in a 50 mM Tris-HCl buffer containing 0.1 M NaCl, 8 mM magnesium sulfate and 0.01% gelatin (pH 7.5, hereinafter referred to as "SM buffer") after a drop of chloroform was added; the mixture was then left at 4°C overnight, to thereby extract the λ phage in the plaques. The operation so far mentioned was repeated until all of the plaques became reactive to the above-mentioned monoclonal antibody. Thus, the DNA coding for the antigen polypeptide was cloned.

As a result, the λ phage was obtained which would express the *C*. *pneumoniae*-specific antigen polypeptide that was reactive to the *C*. *pneumoniae*-specific monoclonal antibody.

### 4.4 Culture of the Obtained λ Phage and Purification of the DNA

Plaques were formed in substantially the same manner as described in 4.3 above. One of those plaques was recovered, placed in 100 µl of SM buffer and left at 4°C overnight, to thereby extract the λ phage. To 250 µl of *E*. *coli* strain Y1090r- which had been incubated in LB culture solution overnight, a solution of the λ phage (5 - 10 µl) was added and left at 37 °C for 20 minutes, to thereby infect the *E*. *coli* with the λ phage. The infected cells were inoculated into LB medium containing 10 mM magnesium sulfate which was warmed to 37 °C in advance, and the cells were incubated under shaking at 37 °C for 5 to 7 hours until lysis of the *E*. *coli* by the λ phage occurred. Then, 250 µl of chloroform was added and centrifuged at 3,000 rpm for 10 minutes to remove the *E*. *coli* cell residue. Thus, a λ phage suspension was obtained. The λ phage DNA was purified using Wizard λ Preps Kit (Promega).

### 4.5 Amplification of the DNA coding for the C. pneumoniae Antigen Polypeptide

In a 600 µl microtube, there were placed 61.5 µl of purified water, 10 µl of 10 × reaction buffer (Tris-HCl buffer, pH 8.3, containing 500 mM KCl, 15 mM MgCl₂ and 0.01% gelatin), 1 µl of 20 mM dNTP, 0.1 µl of the obtained λ phage DNA solution, 1 µl of 20 nM λ gt11 forward primer (Takara Shuzo Co., Ltd.), 1 µl of 20 nM λ gt11 reverse primer (Takara Shuzo Co., Ltd.) and 0.5 µl of AmpliTaq DNA Polymerase, and then 2 to 3 drops of mineral oil were layered at the top. Incubation in a cycle consisting of heatings at 94 °C for 30 seconds, at 55°C for 30 seconds and at 73°C for 2 minutes was repeated 30 times to thereby amplify the DNA. After the completion of the reaction, the reaction mixture was subjected to low-temperature melting agarose gel electrophoresis. The amplified DNA was cut out and purified with Wizard PCR Prep Kit (Promega).

### 4.6 Analysis of the DNA Base Sequence

The DNA base sequences were analyzed by carrying out sequence reactions by the fluorescence-labeled terminator cycle sequence method using Taq DNA polymerase, with the PCR amplified DNA being used as a template, and applying the reaction products to Model 373A DNA Sequencer (Applied Biosystems). The DNA base sequences obtained were edited, ligated and their amino acid translational regions were estimated using a DNA sequencing software "DNASIS" (Hitachi Software Engineering).

As a result, it was found that the obtained DNA of the λ phage coded for the amino acid sequence of the 53 K dalton antigen protein of *C*. *pneumoniae*.

From this, it was found that the monoclonal antibody produced by hybridoma cell lines AY6E2E8 recognized the 53 K dalton antigen protein of *C*. *pneumoniae* as an antigen.

### EXAMPLE 5

### Preparation of Fluorescence-Labeled Antibodies

According to the method of Ivan Lefkovits et al. previously described, the monoclonal antibodies produced by hybridoma cell lines CP-11 and AY6E2E8, respectively, were labeled with FITC.

FITC-Celite (Sigma, Cat. No. F1628) was dissolved in DMSO to prepare a 10 mg/ml solution.

On the other hand, using a NAP-10 column, the buffers having the monoclonal antibodies dissolved therein were replaced with 0.1 M NaHCO₃ buffer (pH 8.2 - 8.6).

To 1.5 ml of the obtained monoclonal antibody solution (monoclonal antibody content: 2 mg), 50 µl of the FITC solution was added and left to stand at 4 °C for 8 hours.

The reaction solution obtained was passed through a PD-10 column equilibrated with PBS. Unreacted FITC was removed using PBS. Thus, the FITC-labeled monoclonal antibody was obtained.

### EXAMPLE 6

### Preparation of Freeze-Dried Formulations

1.9 mg of the FITC-labeled monoclonal antibody, 7.6 mg of Evans blue (Wako Purechemical Co., Ltd.), 1.9 g of bovine serum albumin (GIBCO BRL) and 1.9 ml of 10% NaN₃ (Wako Purechemical Co., Ltd.) were dissolved in PBS (-) (Nissui Pharmaceutical Co., Ltd.) to give a total volume of 100 ml.

The resultant solution was dispensed into 1 ml vials, left to stand at -80°C for 1 hour and then freeze-dried in a freeze-dryer (Iwaki & Co., Ltd.) overnight. Freeze-dried formulations (i.e., CP-11 formulation and AY6E2E8 formulation) were thus obtained.

On the other hand, a combined freeze-dried formulation was prepared in a manner similar to that described above using both of the FITC-labeled monoclonal antibodies derived from hybridoma cell lines CP-11 and AY6E2E8.

### EXAMPLE 7

### Staining of the C. pneumoniae Inclusion Body by the Direct Fluorescent Antibody Technique

The freeze-dried formulations prepared in Example 6 were each dissolved in 2 ml of purified water. On a slide glass upon which *C*. *pneumoniae* strain YK-41 was spotted, 8 µl of the obtained solution was placed so that all spot sites would be covered completely with the solution. Then, the slide glass was placed in a humid box, where a reaction was carried out at 37°C for 30 minutes. The slide glass was washed and air dried. Then, the reaction products were included with 100 µl of an inclusion solution [50% (v/v) glycerol-PBS] and examined with a fluorescence microscope. The results are shown in Table 3.

**Table 3**

| Formulation | Staining Strength* |
|---|---|
| CP-11 formulation | + |
| AY6E2E8 formulation | + |
| Combined formulation | ++ |

| | |
|---|---|
| * Very strong reactivity: ++ Strong reactivity: + | |

As shown in Table 3, either CP-11 formulation or AY6E2E8 formulation exhibited strong reactivity which made the detection of *C*. *pneumoniae* possible. Compared to these single formulations, the combined formulation exhibited very strong reactivity, and with this formulation a very good detection of *C*. *pneumoniae* was possible.

The monoclonal antibody of the present invention which has a specific reactivity with the major outer membrane protein from *C*. *pneumoniae* is useful in detecting *C*. *pneumoniae* specifically, because this monoclonal antibody specifically reacts with the MOMP which is contained abundantly in the microorganism. In addition, a small amount of the antibody will suffice for the detection.

Further, the monoclonal antibody of the present invention which also is characterized by not exhibiting any reactivity to the major outer membrane protein from *C*. *trachomatis* is applicable for distinguishing the infection with *C*. *pneumoniae* from the infection with *C*. *trachomatis* which is very frequently isolated clinically. Thus, this antibody is useful for the accurate diagnosis of *C*. *pneumoniae* infections.

In addition, the labeled antibody of the present invention makes it possible to detect and determine *C*. *pneumoniae* by utilizing the substance labeled therewith. Therefore, this antibody is useful for handling a large quantity of samples quickly in clinical tests.

The monoclonal antibody of the present invention which has a reactivity to the 53 K dalton antigen protein of *C*. *pneumoniae* and which also is labeled is useful for the accurate diagnosis of *C*. *pneumoniae* infections, because this antibody can detect the 53 K dalton antigen protein which is the species-specific antigen of *C*. *pneumoniae*.

The cell lines which produce the above-mentioned monoclonal antibodies are useful for preparing the antibodies continuously and in large quantities. These cell lines are also useful as sources of antibody genes.

The methods of the present invention for preparing monoclonal antibodies using these cell lines are useful for preparing the above-mentioned monoclonal antibodies continuously and in large quantities.

The methods for detecting and determining *C*. *pneumoniae* and the reagents for the detection and determination of *C*. *pneumoniae* of the present invention are useful for detecting and determining *C*. *pneumoniae* in an extremely accurate manner.

The methods for diagnosis of *C*. *pneumoniae* infections and the diagnostic reagents for *C*. *pneumoniae* infections of the present invention are useful for the diagnosis of *C*. *pneumoniae* infections in an extremely accurate manner.

According to the method for preparing monoclonal antibody-producing cell lines of the present invention, it is possible to prepare cell lines that can effectively produce monoclonal antibodies against those proteins the production of monoclonal antibodies against which has been difficult to accomplish by conventional methods. The monoclonal antibodies produced by the antibody-producing cell lines of the present invention are useful in various diagnosis methods and diagnostic reagents for specifically detecting a protein of interest, and they are also useful as medicines.

The above-mentioned antibody-producing cell lines are useful for producing such monoclonal antibodies continuously and in large quantities and are also useful as sources of antibody genes.

The method for preparing monoclonal antibodies using the above-mentioned cell lines is useful for preparing the above-mentioned monoclonal antibodies continuously and in large quantities.

## Claims

1. A monoclonal antibody having a reactivity specific to a Chlamydia pneumoniae-derived major outer membrane protein.

2. The monoclonal antibody of claim 1, having a reactivity with an elementary body of Chlamydia pneumoniae.

3. The monoclonal antibody of claim 1 or 2, having no reactivity with a Chlamydia trachomatis-derived major outer membrane protein.

4. The monoclonal antibody of any one of claims 1-3, which is obtainable from hybridoma cell line CP-6 (FERM BP-5155) or CP-11 (FERM BP-5156).

5. A monoclonal antibody having a reactivity with a 53 KDa antigenic protein of Chlamydia pneumoniae and being labeled.

6. The monoclonal antibody of claim 5, having no reactivity with an inclusion body of Chlamydia trachomatis and Chlamydia psittaci.

7. The monoclonal antibody of claim 5 or 6, which is obtainable from hybridoma cell line AY6E2E8(FERM BP-5154).

8. The monoclonal antibody of any one of claims 1-7, wherein the antibody is labeled.

9. A hybridoma cell capable of producing the monoclonal antibody of any one of claims 1-7.

10. The hybridoma cell of claim 9, which is any one of hybridoma cell line CP-6 (FERM BP-5155), CP-11 (FERM BP-5156) or AY6E2E8 (FERM BP-5154).

11. A method for producing a hybridoma cell that produces a monoclonal antibody having a reactivity specific to a protein, which comprises subjecting an animal to immunization with one of the native and the denatured forms of the protein and to a subsequent booster with the other protein, obtaining an antibody producing cell and fusing the antibody producing cell with a myeloma cell.

12. The method of claim 11, which comprises subjecting the animal to immunization with the native protein and to a subsequent booster with the denatured protein.

13. The method of claim 11 or 12, wherein the denatured protein is obtained by treating the native protein with a detergent or a reductant.

14. The method of any one of claims 11-13, wherein the protein is a major outer membrane protein of Chlamydia.

15. A hybridoma cell which is produced by the method of any one of claims 11-14.

16. A method for producing a monoclonal antibody, which comprises culturing the hybridoma cell of claim 15 and recovering the desired monoclonal antibody.

17. A method for detecting and/or measuring Chlamydia pneumoniae, which comprises using the monoclonal antibody of any one of claims 1-8 or a combination thereof.

18. A reagent for detecting and/or measuring Chlamydia pneumoniae, which comprises the monoclonal antibody of any one of claims 1-8 or a combination thereof.

19. A method for diagnosing Chlamydia pneumoniae infection, which comprises using the monoclonal antibody of any one of claims 1-8 or a combination thereof.

20. A diagnostic agent for a Chlamydia pneumoniae infection, which comprises the monoclonal antibody of any one of claims 1-8 or a combination thereof as an active ingredient.


SEQUENCE LISTING

(1) GENERAL INFORMATION:

(i) APPLICANT:
(A) NAME: Hitachi Chemical Company, Limited
(B) STREET: 1-1, Nishishinjuku 2-chome, Shinjuku-ku
(C) CITY: Tokyo
(E) COUNTRY: Japan
(F) POSTAL CODE (ZIP): 163-04

(ii) TITLE OF INVENTION: Monoclonal antibodies having a reactivity
specific to chlamydia pneumoniae, method for production of
the monoclonal antibodies, such antibody producing cells,
method for production of the cells, method for detection
and/or measurement of chlamydia pneumoniae, reagents for
the method, method and agents for diagnosis of chlamydia
pneumoniae infection

(iii) NUMBER OF SEQUENCES: 2

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(v) CURRENT APPLICATION DATA:
APPLICATION NUMBER: EP 95 11 2207.6

(vi) PRIOR APPLICATION DATA:
(A) APPLICATION NUMBER: JP 182240/1994
(B) FILING DATE: 03-AUG-1994

(vi) PRIOR APPLICATION DATA:
(A) APPLICATION NUMBER: JP 182241/1994
(B) FILING DATE: 03-AUG-1994

(vi) PRIOR APPLICATION DATA:
(A) APPLICATION NUMBER: JP 264307/1994
(B) FILING DATE: 28-OCT-1994

(vi) PRIOR APPLICATION DATA:
(A) APPLICATION NUMBER: JP 141721/1995
(B) FILING DATE: 08-JUN-1995

(2) INFORMATION FOR SEQ ID NO: 1:

(i) SEQUENCE CHARACTERISTICS:
(A) LENGTH: 16 base pairs
(B) TYPE: nucleic acid
(C) STRANDEDNESS: single
(D) TOPOLOGY: linear

(xi) SEQUENCE DESCRIPTION: SEQ ID NO: 1:

AATTCGAACC CCTTCG 16

(2) INFORMATION FOR SEQ ID NO: 2:

(i) SEQUENCE CHARACTERISTICS:
(A) LENGTH: 12 base pairs
(B) TYPE: nucleic acid
(C) STRANDEDNESS: single
(D) TOPOLOGY: linear

(xi) SEQUENCE DESCRIPTION: SEQ ID NO: 2:

CGAAGGGGTT CG 12